# EUROPEAN PATENT APPLICATION

(11) **EP 1 676 583 A2**
(43) Date of publication of application: **05.07.2006**
(21) Application number: 04792620.9
(22) Date of filing: 13.10.2004
(51) Int. Cl.: A61K 35/56

(54) **THERAPEUTIC AGENT FOR PERIODONTAL DISEASE**

(30) Priority: 16.10.2003 JP 2003356425
(71) Applicant: CHAFFLOSE CORPORATION, Yokohama-shi, Kanagawa 231-0034 (JP)
(72) Inventor: KOYAMA, Nobuji, Hachinohe-shi, Aomori 031-0813 (JP); OKUDA, Shinichi, Sendai-shi, Miyagi 980-0801 (JP); SASAYA, Koji, Chafflose Corporation, Yokohama-shi, Kanagawa 231-0034 (JP); YOSHIDA, Tomonaga, Hachinohe-shi, Aomori 031-0812 (JP)
(74) Representative: Leitner, Waldemar
(86) International application number: PCT/JP2004/015454
(87) International publication number: WO 2005/037188

(57) **Abstract**

A therapeutic agent for periodontal diseases is provided which is prepared by utilizing a scallop shell material and which has antimicrobial activity against periodontal disease-inducing bacteria, promotes the regeneration of the alveolar bone and enables adequate prevention and treatment of periodontal diseases. The therapeutic agent for periodontal diseases is obtained from an aqueous solution prepared by dissolving, in water, a mixture of a calcium carbonate powder prepared by subjecting shells having a crystal structure comprising a calcite type structure of calcium carbonate to grinding and primary firing, and a calcium oxide powder prepared by subjecting the calcium carbonate powder to firing at a temperature higher than the primary firing temperature.

## Description

### FIELD OF THE INVENTION

This invention relates to a therapeutic agent for periodontal diseases.

### DESCRIPTION OF THE PRIOR ART

Periodontal diseases are diseases around the teeth as caused by plaque (mass as a nest of microorganisms) on the tooth surface and roughly classified into gingivitis characterized by hemorrhage and swelling due to inflammation of the gingiva (gum), and periodontitis characterized by destruction of the alveolar bone which supports the teeth. Since periodontal diseases are caused by causative bacteria whose nest is the above-mentioned plaque, the importance of brushing teeth daily for removing plaque has so far been emphasized. Under the existing circumstances, however, there are many cases of periodontal diseases as a result of inadequate removal of plaque. Under these circumstances, therapeutic agents for periodontal diseases or tooth pastes or the like containing such a substance having antimicrobial activity against periodontal disease bacteria as a crude drug extract have been proposed (cf. e.g. Patent Document 1: Japanese Kokai Publication No. 2003-081800; Patent Document 2: Japanese Kokai Publication No. 2001-294536).

### SUMMARY OF THE INVENTION

The conventional therapeutic agents for periodontal diseases, such as those mentioned above, are allegedly intended to treat such diseases by the antimicrobial activity against such bacteria as Prophyromonas gingivalis. However, when the antimicrobial activity alone is relied on against the symptoms at a stage at which the damaging of the alveolar bone has already begun, the progress of alveolar bone damaging may be inhibited but the regeneration of the alveolar bone cannot be assisted; only the regenerative power on the side of the human body by means of blood circulation stimulation in the gingiva and activation of osteoblasts, for instance, can be hoped for.

On the other hand, the present inventor paid attention to the fact that a powder obtained by firing the scallop shell so far disposed of as a useless matter, followed by grinding, and a water-soluble fraction thereof show alkalinity and the fact that such powder and water-soluble fraction have natural product-due antimicrobial activity. Thus, in view of the above-discussed state of the art, it is an object of the present invention to make it possible to adequately prevent and treat periodontal diseases using a therapeutic agent for periodontal diseases in which a scallop shell-derived material is utilized and which has antimicrobial activity against those bacteria inducing periodontal diseases and can promote the regeneration of the alveolar bone.

The present invention, which has been made to accomplish the above object, solves the problems mentioned above by providing a therapeutic agent for periodontal diseases which is characterized in that it is an aqueous solution obtainable from dissolution, in water, of a mixture of a calcium carbonate powder prepared by subjecting shells having a crystal structure comprising a calcite type structure of calcium carbonate to grinding and primary firing and a calcium oxide powder prepared by subjecting the above-mentioned calcium carbonate powder to firing at a temperature higher than the primary firing temperature.

The therapeutic agent according to the invention contains a nature-derived calcium carbonate powder dissolved therein, and the nature-derived antimicrobial calcium carbonate shows good bactericidal and eradicative activities against periodontal disease bacteria and, thus, the therapeutic agent can be used in the prevention and treatment of periodontal diseases. Further, the whole water-soluble fraction is made from a natural material and is free of any chemically synthesized substance and thus is advantageous in that it does not exert any adverse effect on the health of patients with periodontal diseases.

Furthermore, since scallop shells are used as the raw material, the scallop shells so far mostly discarded as a useless matter can be efficiently used. The constitution is simple and the production process is not complicated and, therefore, the agent can be provided at low cost.

Upon application of the therapeutic agent for periodontal diseases of the invention, the diseased site is neutralized and the activation of periodontal disease bacteria, odontoclasts and osteoclasts is thereby prevented, and the regeneration of the alveolar bone is promoted by the calcification resulting from application of the therapeutic agent for periodontal diseases. Thus, the agent produces excellent effects from the practical viewpoint.

### DETAILED DESCRIPTION OF THE INVENTION

The present invention is now described in detail referring to a typical mode of embodiment thereof.

For preparing the therapeutic agent for periodontal diseases of the invention, first, scallop shells at least the major portion of which has a crystal structure comprising a calcite type structure of calcium carbonate are prepared. While there are various shell species, the shells of bivalves, in particular scallops, are characterized in that the major portion of each shell has such crystal structure.

Ecologically, scallops, for instance, are characterized by their behavior in quickly moving by opening and closing the shell to discharge seawater vigorously toward the outside, as expressed by the phrase "swim in the sea", and thus escaping from predators (starfish etc.). They have a large ligament to enable such movement and, further, the shell itself is relatively thin and light in weight and is tough and strong. In that shell, the shell inside surface side is constituted of a crystal structure of the calcite type structure of calcium carbonate which forms a lamellar structure and, in the shell inside layer (layer serving as the core in the direction of the thickness of the shell), a crystal structure of the calcite type of calcium carbonate forms a sheet-like structure. These shell inside surface side and shell inside layer constitute the main part of the shell (excluding the surface layer and hinge portion) and, therefore, the main part is tough and strong in spite of its being thin and light in weight.

In the main part of the scallop shell, the inside surface side has a lamellar structure (the structure that needle crystals are compactly spread and aligned all over in the manner of a needle point holder) consisting of a crystal structure of the calcite type structure of calcium carbonate, and the shell inside layer has a sheet-like structure consisting of a crystal structure of a calcite type structure of calcium carbonate (the structure that each layered needle crystals are arranged in the same direction, overlapping, like plywood, with the direction of the needle crystals differing from layer to layer), as mentioned above, so that the grains obtained by grinding the shell of scallops, which are bivalves, retain the calcite type structure and has porosity, as described later herein. On the contrary, the shells differing in morphology have a structure resulting from overlapping of planes of calcium carbonate crystals spreading therein and, owing to this, show a pearly luster; however, the granular bodies obtained therefrom have no porosity.

In preparing the therapeutic agent for periodontal diseases, the above-mentioned scallop shells were used as the raw material. The scallop shells that have been discarded as a useless material after removal of the ligament can now be utilized; effective utilization of the waste matter is thus possible. First, shells no more required are collected, and dried and hardened on exposure of the sun. Then, the shells hardened by drying in the sun are placed in a container and, after evacuating the inside of container to be free of oxygen, the contents are fired at a temperature of about 200°C (primary firing), followed by grinding to a grain diameter of about 200 µm. The method of grinding itself is not particularly restricted but any of the existing grinding apparatus can be used. In this way, a calcium carbonate powder is obtained; the granular bodies thereof are porous.

Then, a portion of the calcium carbonate powder consisting of the porous granular bodies mentioned above is used and placed in a container and, after evacuating the inside of the container to be free of oxygen, is fired by heating at a temperature of about 800°C for about several hours (secondary firing) to give a calcium oxide powder. The calcium oxide powder thus obtained after secondary firing is mixed with the calcium carbonate powder obtained by primary firing in a mixing ratio of 1:1 (by weight). The thus-obtained mixture is further mixed with water in a mixing ratio of 1:9 (by weight) to give an aqueous solution resulting from dissolution of the mixture in water. An aqueous solution containing calcium hydroxide and having a pH of about 9 is thus obtained, and this is the therapeutic agent for periodontal diseases.

As mentioned above, the above therapeutic agent for periodontal diseases is a product obtained using the scallop shell powder, which is a natural product, as the raw material, and this therapeutic agent for periodontal diseases itself has antimicrobial activity as a natural product. The calcium carbonate powder obtained after primary firing, in particular, has good antimicrobial activity as a nature-derived product, and the agent displays the above-mentioned antimicrobial activity owing to its containing this calcium carbonate dissolved therein. The nature-derived product, so referred to herein, is oppositely positioned to chemically synthesized products, and therefore the shells of farmed scallops are also to be regarded as a nature derived product. The therapeutic agent for periodontal diseases as a whole shows alkalinity and, owing to its alkaline physical property, it shows high antimicrobial activity.

Thus, the present therapeutic agent for periodontal diseases has both natural product-due antimicrobial activity and alkalinity-due antimicrobial activity and thus has an effect of inhibiting the growth of microorganisms and actively inhibits the growth of periodontal disease bacteria. As for the usage, it is only necessary to apply this therapeutic agent for periodontal diseases to the gingival portion, whereby periodontal disease bacteria can be killed.

The effects of the therapeutic agent for periodontal diseases were verified by the following experiment.
(1) First, 0.9 ml of the aqueous scallop shell ceramics solution (therapeutic agent for periodontal diseases of the invention) was placed in a sterilized microtube and, after addition thereto of 0.1 ml of a culture of periodontal disease bacteria, this microtube was incubated in a constant-temperature bath maintained at 30°C for 3 minutes (time period recommended for teeth brushing).
(2) The aqueous scallop shell ceramic solution after treatment as described above under (1) was streaked on a blood agar plate medium for the culture of periodontal disease bacteria, and the bacteria were cultured under anaerobic environment conditions at 37°C for 4 days.

In a comparative example, an experiment was carried out in the same manner as described above using physiological saline in lieu of the aqueous scallop shell ceramics solution.

### (Results of the experiments)

When periodontal disease bacteria were treated with physiological saline and then cultured at 37°C for several days (comparative example mentioned above), the growth of periodontal disease bacteria was confirmed. On the contrary, no growth of periodontal disease bacteria was observed at all in the case of treatment with the aqueous scallop shell ceramics solution. Thus, it can be judged that the aqueous scallop shell ceramics solution, namely the therapeutic agent for periodontal diseases according to the invention, has antimicrobial activity against periodontal disease bacteria.

When this therapeutic agent for periodontal diseases is applied to the site suffering from periodontitis, the surface layer of the site of periodontitis on the alveolar bone, among others, is dissolved by the action of the alkalinity of the calcium hydroxide component in this therapeutic agent for periodontal diseases and, then, the proliferation of capillary blood vessels occurs there, the calcium ions transported by the blood running through the newly formed capillary blood vessels and the calcium ions contained in the present therapeutic agent for periodontal diseases react with carbon dioxide or the like in the tissue or air to form a calcium carbonate layer and, thereby, the phenomenon of calcification is induced, the site of periodontitis on the alveolar bone is therapeutically treated and the regeneration of the alveolar bone is promoted.

When, at the site of periodontitis, the inflammatory lesion acquires acidity, periodontal disease bacteria, odontoclasts and osteoclasts are activated. However, when the site of inflammation is neutralized by the application of the present therapeutic agent for periodontal diseases, as mentioned above, the above-mentioned activation of periodontal disease bacteria, odontoclasts and osteoblasts is inhibited. Furthermore, the agent is effective in inducing the regeneration of the alveolar bone owing to the calcification mentioned above.

The agent according to the invention contains a nature-derived calcium carbonate powder dissolved therein and, the antimicrobial activity of the natural product calcium carbonate exerts satisfactory bactericidal and eradicative actions on periodontal disease bacteria and, thus, the agent can be used in the prevention and treatment of periodontal diseases. Further, the water-soluble fraction as a whole is free of any chemically synthesized substance and is purely of natural material origin. This is advantageous since the health of the patient is never adversely affected.

Further, since scallop shells are used as the raw material, the scallop shells so far disposed of as a useless material can now be efficiently utilized. The agent is simple in constitution and, therefore, no complicated production process is needed, hence it can be provided at low cost.

## Claims

1. A therapeutic agent for periodontal diseases which comprises an aqueous solution obtainable from dissolution, in water, of a mixture of a calcium carbonate powder prepared by subjecting shells having a crystal structure comprising a calcite type of structure of calcium carbonate to grinding and primary firing, and a calcium oxide powder prepared by subjecting the calcium carbonate powder to firing at a temperature higher than the primary firing temperature.
